# EUROPEAN PATENT APPLICATION

(11) **EP 0 612 844 A2**
(43) Date of publication of application: **31.08.1994**
(21) Application number: 94301315.1
(22) Date of filing: 24.02.1994
(51) Int. Cl.: C12N 15/11, C12N 9/00, A61K 31/70, C12N 15/86, C12N 15/12, C12N 7/01, C12N 5/10, C12N 15/87

(54) **Expression constructs containing HIV inhibitory antisense and other nucleotide sequences, retroviral vectors and recombinant retroviruses containing same**

(30) Priority: 25.02.1993 US 21936; 01.02.1994 US 190350
(71) Applicant: ORTHO PHARMACEUTICAL CORPORATION, Raritan, NJ 08869-0602 (US)
(72) Inventor: Pyati, Jagdeesh, San Diego, CA 92129 (US)
(74) Representative: Fisher, Adrian John

(57) **Abstract**

This invention relates to the treatment of disease states, and especially AIDS, through the administration of antisense and other nucleotide constructs. The constructs are administered to the patient in a traditionally pharmaceutical manner, or through the use of recombinant retrovirus delivery systems. The latter delivery systems preferably target specific cell types or other components one desires to target. Such targeting is accomplished by modifying the envelope of a recombinantly produced therapeutic retrovirus to contain ligand (receptor) sequences for which a receptor (ligand) exists on the cell type or other component in question.

## Description

### CROSS REFERENCE TO RELATED APPLICATION DATA

This application is a continuation-in-part of copending U.S. application Serial No. 08/021,936, filed February 25, 1993.

### FIELD OF THE INVENTION:

This invention relates to the treatment of disease states, and especially AIDS, through the administration of antisense and other nucleotide constructs. The constructs are administered to the patient in a traditionally pharmaceutical manner, or through the use of recombinant retrovirus delivery systems. The latter delivery systems preferably target specific cell types or other components one desires to target. Such targeting is accomplished by modifying the envelope of a recombinantly produced therapeutic retrovirus to contain ligand (receptor) sequences for which a receptor (ligand) exists on the cell type or other component in question.

### BACKGROUND OF THE INVENTION:

A myriad of approaches are currently being investigated for treatment of viral infections and other disease states. One of the newest approaches in treating viral infections is to use antisense oligonucleotides to selectively block virus replication, undesired protein production, and the like, usually by selective, competitive, hybridization. For example, Goodchild et. al. in Proc. Natl. Acad. Sci. Vol.85, pp. 5507-5511 reports on studies to inhibit HIV replication by the use of certain antisense oligonucleotides, targeted to twenty different target sites within human HIV RNA. The target sites were selected based upon their potential capacity to block functions during viral replication. The oligos were about 20 to about 12 base pairs in length. Some of the oligos were able to demonstrate an inhibitory effect, but this varied greatly between the various constructs. None of them stood out as particularly superior to the rest, and in fact, the oligos may be too small to effectively inhibit all of the areas necessary to achieve acceptable inhibition of the virus.

Retroviral vectors used as delivery systems to deliver genetic information, including antisense, have been suggested to treat nongenetic diseases as well as genetic diseases. Tellier et al., Nature 318:414, 1985, suggested protecting T-cell clones by infecting stem cells with "defective" HIV, which has a genome that expresses antisense RNA, complementary to HIV RNA. However, it appears that this recombinant retroviral construct could actually reproduce like a wild-type virus and hence, cannot be considered "replication" defective. Using the Tellier techniques, newly constructed retroviruses could infect other cells, causing an increased risk of recombination with previously present HIV or other nucleotide sequences. This leads to loss of control and possible generation of undesirable new recombinant sequences, whose replication go unchecked throughout the patient.

Hence, there remains a need for better therapeutic and immunizing protocols to treat viral infections, and the like, using more effective nucleotide constructs which can be delivered to the target of interest in an efficient, safe manner. The present invention, as will be described in detail herein, provides several advantages over the art in this regard. The therapeutic targeting embodiments of the invention are very efficient, and therefore, relatively lower titers of the recombinant retrovirus are needed. Secondly, in some embodiments, the recombinant virus is delivered only to a prechosen cell type, and thus, virus infects only target cells and is incapable of infecting other cell types, eliminating unnecessary widespread cellular infection.

The advantages of incorporating antisense polynucleotide sequences, such as those provided herein, into the retroviral vector embodiments of the invention, are many. In accordance with a prior art method of direct injection of antisense oligonucleotides, one needs a repeated, exogenous supply of oligos, which invariably have to be modified, to extend their half-life once injected into the patient. By contrast, a single infection of a cell by a recombinant retrovirus will enable a continuous supply of antisense RNA to be produced inside the susceptible cell. Long stretches of these antisense sequences may be cloned into a vector, increasing the chances of specificity for the target. By contrast, long stretches of free oligos cannot enter cells, and therefore, shorter stretches are generally chosen. This has engendered new problems in the art, in that shorter stretches of oligonucleotides freely enter a random population of cells. The drawbacks inherent with this random entering into these cells is that large amounts of these oligos are then required to ensure that the cells it is desired to target are exposed to the oligos. This is in addition to the fact that it may be undesirable that a random population of cells are non-specifically affected in this manner in the first place.

### SUMMARY OF THE INVENTION:

The present invention provides antisense and other nucleotide Expression Constructs, preferably in combination with a recombinant retroviral delivery system (Retroviral Vector and/or Recombinant Retrovirus) that targets specific cell types to infect such cells, and to deliver the inhibitory antisense sequence, or other sequences it is desired to express. In preferred embodiments, the Recombinant Retrovirus is substantially incapable of reproducing in the cells so infected. Typical examples of targeting as described more fully herein are: A recombinant retrovirus with gp120 on the envelope so that the vector may be targeted to CD4+ cells. Conversely, the envelope may contain CD4, so that the vector may be delivered to gp120+ (HIV harboring) cells.

In these preferred retroviral delivery system embodiments, and upon infection of the target cell, a Retroviral Vector, containing the Expression Construct of interest, is delivered into the cell. The nucleotide information carried by the Expression Construct may be copied into DNA, at least a portion of which may then integrate as a DNA provirus into the cellular genome. Transcription of the Expression Construct now present in the provirus form (under the control of an appropriate promoter) produces RNAs which may be translated into proteins. If the Retroviral Vector contains an Expression Construct that expresses an antisense polynucleotide sequence, then RNA antisense (complementary) to portions of the RNA of virus that one desires to inhibit is produced. The RNA that is inhibited may be in the form of mRNA produced by infected cells, full-length genomic RNA produced by infected cells, or viral genomic RNA first delivered to the cell upon infection with a virion particle. The antisense RNA binds to the target RNA and inactivates it. In this manner, "molecular immunity" may be conferred upon the cells.

In preferred embodiments, one or more ribozyme nucleotide sequence, acting as genetic "shears", may be interspersed among the antisense sequences. In that case, the antisense RNA will readily recognize target RNA and bind to it and the ribozyme (s) will cleave it.

The Retroviral Vector may also contain a gene or portion thereof whose protein product one desires to express in target cells. Such proteins may be confined inside the cells, expressed on the cellular surface, or secreted out of the cells. In a preferred embodiment, soluble CD4 is the protein product.

Further aspects of the invention concern cells that have been transformed with the Expression Constructs of the invention, and especially the Retroviral Vectors as delivered by Recombinant Retroviruses, with the result that a disease state is alleviated, or resistance to such disease state is conferred. Methods of producing Expression Constructs, Retroviral Vectors, Recombinant Retroviruses, Producer Cells, foreign RNA or other nucleotide sequences of interest, and methods for producing a polypeptide, transiently or constituitively are also provided. Therapeutic treatment, including immunization methods using the Expression Constructs, Recombinant Vectors and Recombinant Retroviruses are also provided herein.

### BRIEF DESCRIPTION OF THE FIGURES:

Figure 1 is a schematic representation of certain Expression Construct embodiments of the invention as derived from the HIV genome.

Figure 2 is a polynucleotide sequence relating to the Expression Construct HXB5 (SEQ. ID NO:1)

Figure 3 is a polynucleotide sequence relating to the Expression Construct RZenv1 (SEQ. ID NO:2).

Figure 4 is a polynucleotide sequence relating to the combination of HBX5 and RZenv1 (SEQ. ID NO:3).

Figure 5 is a schematic representation of a hammerhead ribozyme nucleotide sequence.

Figure 6 is a schematic representation of the cloning of certain Expression Construct embodiments into a retroviral vector.

Figure 7 is a schematic representation of the construction of a packaging cell line wherein the envelope portion of such packaging cell bears gp120, rendering a CD4 tropic Recombinant Retrovirus.

Figure 8 is a schematic representation of expression of the envelope portion of HIV (gp120) in the pEE6 expression vector.

Figure 9 is a schematic representation of expression of CD4 in the pEE6 expression vector.

Figure 10 is a schematic representation of construction of a packaging cell line selective for cells harboring the HIV virus (expressing gp120).

Figure 11 is a schematic representation of a CD4 tropic packaging cell testing cell line.

Figure 12 is a schematic representation of a gp120 tropic packaging cell testing cell line.

Figure 13 is a schematic representation of a gp120 tropic Recombinant Retrovirus of the invention.

Figure 14 is a schematic representation of insertion of the CD4 ligand into a vector for preparation of the gp120 tropic packaging cell line in accordance with the teachings herein.

Figure 15 is a polynucleotide sequence relating to the Expression Construct HXB5 of a variant strain of HIV (SEQ. ID NO:4)

Figure 16 is a polynucleotide sequence relating to the Expression Construct RZenv1 of a variant strain of HIV (SEQ. ID NO:5).

Figure 17 is a polynucleotide sequence relating to the combination of HBX5 and RZenv1 of a variant strain of HIV (SEQ. ID NO:6).

### DETAILED DESCRIPTION OF THE INVENTION:

### Definitions:

As used herein, "substantially complementary" refers to nucleotide base pair binding of a nucleotide sequence to a target nucleotide sequence to such an extent that the transcriptional or translational activity of such target sequence is effectively retarded or inhibited.

As used herein, "polynucleotide" means a relatively small natural or synthetic nucleic acid polymer containing from about 2000 to about 200 nucleotide bases.

As used herein, "transformation" means the process of changing the genotype of a recipient cell mediated by the introduction of genetic information contained in the Expression Constructs of the invention.

As used herein, a "polyadenylation splicing (or addition) site" is a DNA sequence located downstream from the translated regions of a gene, enabling adenine ribonucleotides to be added to form a polyadenine nucleotide tail at the 3' end of messenger RNA. Polyadenylation is important in the stabilization of messenger RNA against degradation in the cell, an event that reduces the level of messenger RNA and the level of product protein. It is sometimes referred to herein as (A)n.

As used herein, "LTR" refers to long terminal repeat sequences, which are the structural elements that provide for the integration of DNA copied from a retroviral RNA into the genomic DNA of the cells infected by the retrovirus, in the form of a provirus. 3' LTR is the LTR situated on the 3' end of the sequence, with 5' LTR being the LTR upstream from the 3' end, otherwise referred to as the 5' end.

As used herein, "provirus" means the double-stranded counterpart of the retroviral genome that comprises an essential stage in the life cycle of a retrovirus.

As used herein, " Expression Construct" means a recombinant assemblage comprising nucleotide sequences of interest, especially in antisense or endoribonuclease formation, or combinations thereof, and when so oriented, at least a portion of which is substantially complementary to a target RNA sequence, said assemblage useful to inhibit the transcriptional or translational activity of said target sequence.

As used herein, "Retroviral Vector" means a vehicle that includes retroviral nucleotide sequences and is intended for introduction of genetic material such as the Expression Constructs of the invention, into a cell, and to make use of the cell's synthetic machinery in the manner of retroviruses.

As used herein, "Recombinant Retrovirus" means a recombinant assemblage containing a Retroviral Vector in a packaging cell line rendering a virion particle that is capable of infecting cells, in the manner of virion particles, to deliver genetically engineered material, but is replication defective in that it is substantially incapable of packaging its engineered genomic material into new virion particles that would then bud from the cells, and infect new cells.

As used herein "Producer Cells" means cells or a cell line that produce or express the Expression Constructs, the Retroviral Vectors, or the Recombinant Retroviruses (virion particles).

As used herein, "Stably Transformed Cell" means a cell wherein the Expression Constructs and/or Retroviral Vectors and/or Recombinant Retroviruses have integrated into the cell and have induced the cell to express foreign RNA, DNA, endoribonucleases, other nucleotide sequences of interest, or protein products.

In a first embodiment of the invention, certain Expression Constructs are provided, which comprise nucleotide sequences of interest. In a preferred Expression Construct embodiment, an anti-sense Expression Construct is provided, which, when introduced into a cell, and even an HIV infected cell, is capable of directing the transcription of RNA complementary to RNA encoding a portion of the HIV genome. While the present inventor does not wish to be bound by theory, it is contemplated that the anti-sense Expression Construct thus directs the transcription of "anti-sense" RNA which is capable of hybridizing to all or a part of the endogenous HIV viral RNA or plus strand DNA in a eucaryotic host cell or the mRNA transcribed from the HIV proviral DNA. This in turn can work to prevent or decrease (preferably significantly) the activity of HIV RNA, whatever the source, and effective replication of the HIV virus. In one instance, the viral RNA that is delivered to the cells by virion particles and then enters these cells previously protected with the Expression Constructs, is substantially prevented from its reverse transcription into DNA, and hence, cannot effectively integrate into its host's genome. In another instance, the plus strand DNA which is complementary to the minus strand DNA transcribed from this genomic viral RNA, may also be inhibited by binding to the Expression Construct. In yet another instance, the level of transcription of the HIV genome into viral mRNA and the translation of the mRNA into HIV viral proteins by the cell is greatly interfered with, and even inhibited completely. In a fourth instance, the production and successful packaging of new viral genomic RNA, meant for new virion particle packaging, is effectively interfered with. The overall result is that HIV virus that has already gained entry to the cell, or does gain entry, fails to adequately take over the cell's synthetic machinery and replicates substantially less or even fails to replicate at all. In more preferred embodiments, at least about 70% and more preferably at least about 80% inhibition of such viral production is conferred upon cells transformed or transfected with the anti-sense Expression Constructs of the invention. In certain other preferred embodiments, one or more ribozyme nucleotide sequences may be interspersed among the antisense sequences of the invention. When the antisense binds, the ribozyme goes to work cleaving the target RNA. This action enhances the possibility of substantially inhibiting the would be activities of the HIV RNA. In cells that were previously harboring latent HIV provirus, and in which the provirus is activated to make viral RNA, inhibition of such viral RNA blocks effective packaging of the HIV viral RNA into viral particles that bud from the cell to infect other cells. The inhibition of the HIV RNA may also confer molecular immunity upon the cells. Uninfected cells susceptible to HIV infection may be protected from eventual HIV infection, as the viral genome is prevented from transcription into a provirus and integration into the genome of it's host.

By way of further exemplification of the present invention, certain preferred Expression Constructs of the invention will be set forth in greater detail. Antisense constructs directed to various structural or other portions of the HIV genome as exemplified in the Examples section, were constructed by restriction and ligation of genomic fragments, and then, synthesis of doubled-stranded DNA to obtain the antisense strands. Those skilled in the art readily understand that certain references are available, publishing the sequence of the HIV genome. From this information, one may deduce the areas of the genome useful for the purposes of creating antisense constructs in accordance with the teachings herein. Preferred constructs of the invention comprise an antisense sequence substantially complementary to various portions of HIV genomic RNA. The HIV genomic fragments are obtained through the use of various restriction enzymes, such as EcoR1, EcoRV, HindIII+PstI, BamH1, XbaI+NheI, and the like, depending on the areas of the genome it is desired to fragment. All fragments may be obtained, and all further recombinant constructions may be prepared using standard recombinant techniques. See generally T. Maniatis et. al., Molecular Cloning: A Laboratory Manual, 2nd edition, Cold Spring Harbor Press, 1990. Selection of the appropriate coordinates is in accordance with the numbering set forth in RNA Tumor Viruses, Vol. II. ed. by Weiss et. al. 1985 - Cold Spring Harbor Laboratory and Ratner et al., Nature 313:277-284 (1985).

A suitable starting material for construction of fragments, to which complementary sequences can be produced is the plasmid pHXB2D, which comprises HIV proviral sequences cloned into the plasmid SP62. This may be obtained from Robert Gallo's laboratory at the National Institute of Health, In Bethesda, Maryland, U.S.A. pHXB2D may be cut with EcoR1 + EcoRV to obtain genomic fragments, which may be inserted into various expression vectors in the antisense orientation.

Although these techniques were chosen for preparation of the constructs, one skilled in the art will understand that the Expression Constructs of the invention may be synthesized de novo in accordance with well-accepted nucleic acid polymer synthesis techniques, by merely referring to the sequence and related information disclosed herein, and simply deducing the complementary sequence useful to prepare the Construct. One suitable method of nucleotide synthesis is the phosphite triester method using an Applied Biosystems Model 380A DNA synthesizer, using standard chemistries as recommended with such a synthesizer (Matteuchi, et. al., Journal Amer. Chem. Soc. 103:3185-3319, 1981). The crude polynucleotide mixture may be purified by gel electrophoresis, and the like.

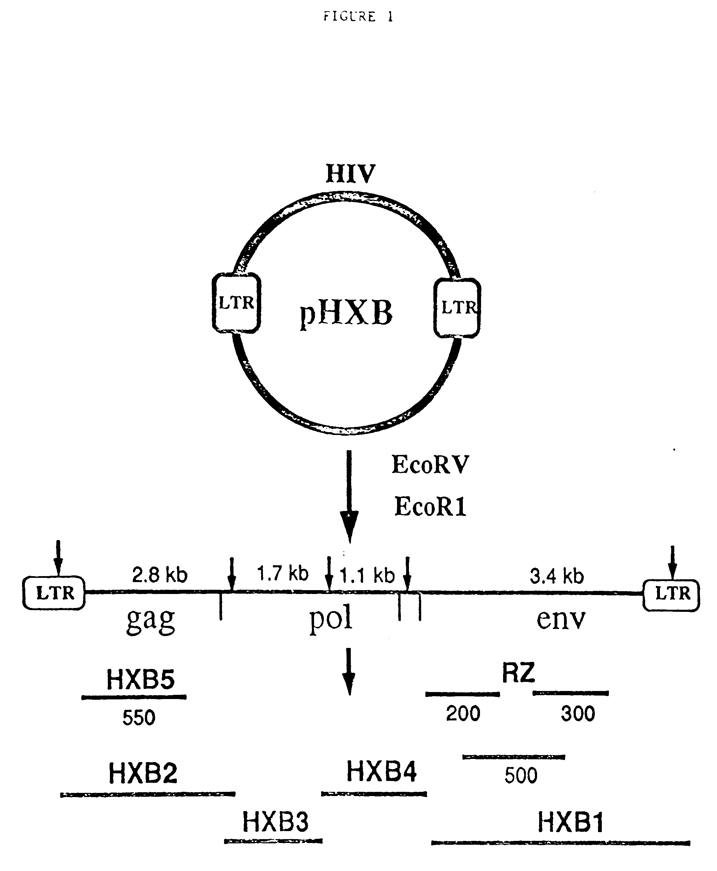

Preferred fragments are shown in Figure 1. As depicted in Figure 1, a useful cDNA fragment corresponds to at least a portion of the env region of the HIV genome, which extends for about 3.4kb from the 3' LTR of the HIV viral genome (nts 5322-8694). This fragment, when expressed or synthesized in the antisense orientation, has been designated Expression Construct "HXB1". A second useful construct is complementary to a fragment that corresponds to a region about 2.8kb downstream from the 5' LTR of the HTV genome, in the gag area (nts -342-2556). This Expression Construct has been designated "HXB2". A particularly preferred Expression Construct is complementary to the pol region of the HIV genome extending from about 2.8kb to about 4.5kb downstream from the 5'LTR of the virus genome (nts 2556-4227). This has been designated "HXB3". More preferred is a construct comprising an antisense sequence substantially complementary to the HIV genome in the pol and tat region, about 1.1kb in length and situated from about 4.5kb to about 3.4kb upstream from the 3' LTR of the HIV viral genome (nts 4227-5322). This Expression Construct is designated "HXB4".

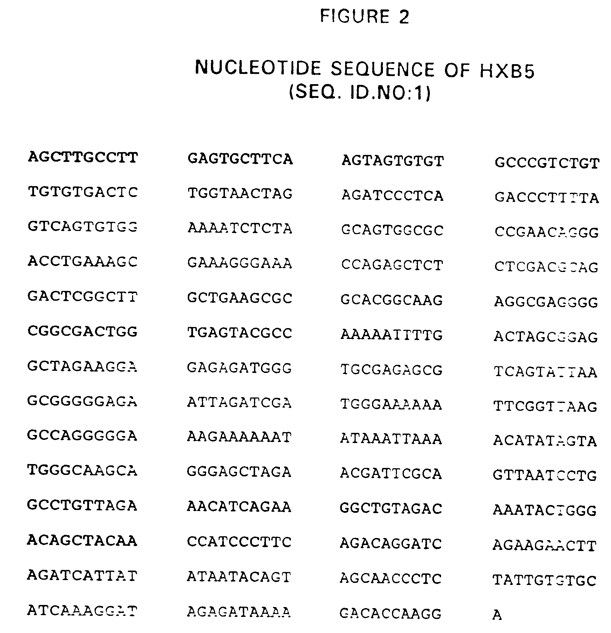

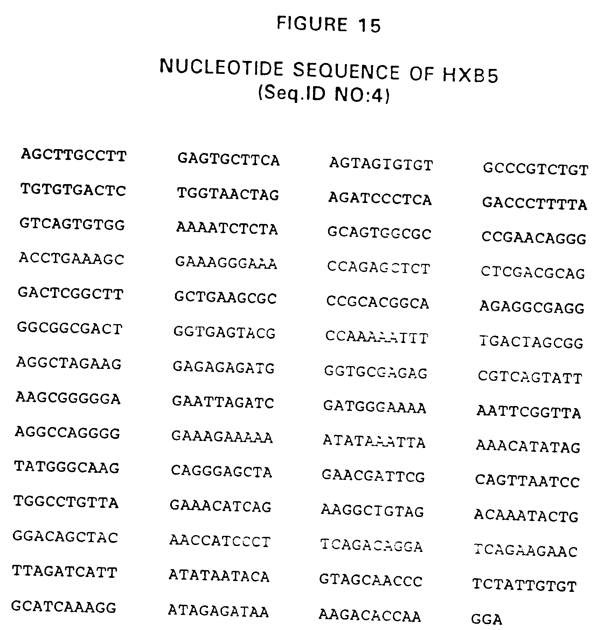

A particularly preferred subconstruct corresponding to about 550bp (nts77-628) of HXB2 (the sequence is set forth in Figure 2, or a variant thereof, the sequence set forth in Figure 15), has also been created and is designated "HXB5" (SEQ. ID NO:1), or , depending on the HIV strain, is that shown designated SEQ. ID NO:4. The HIV genomic region this construct is complementary to when in antisense orientation are portions that span the R, U5, and 5' part of gag. Important regions are the leader sequences, the Primer Binding Site (PBS), the packaging signal, the Splice Donor (SD), and gag start codon. The construct is provided in antisense orientation so that it is substantially complementary to the aforementioned regions of the HIV genome.

Also particularly preferred for use herein are chimeric Expression Constructs which contain two or more nucleotide sequences, which when provided in antisense orientation, are complementary to non-contiguous segments located at various places in the HIV genome. The two or more antisense segments may flank other nucleotide sequences, such as for example, a ribozyme sequence, or some other sequence of interest that may aid in the intended result of destruction or inhibition of protein expression, viral replication, and other genetic expression it is desired to effect or inhibit. It is within the scope of the invention that this flanking effect could be repeated, so that more than one other sequence of interest is flanked by antisense segments, or the same effect is repeated one or more times. This can result in a construct that has, for example, multiple ribozyme areas, flanked by antisense areas. The antisense segments, when used in combination, such as in the manner just described, are preferably less than about 1kb because the likelihood of genetic recombination to form a sense strand is reduced; segments smaller than about 1kb are generally easier to express; and the likelihood of expression of undesirable structural regions is minimized. Further, the combination of antisense segments to multiple regions in the HIV genome allows better accuracy in targeting regions that are important in the overall inhibition of HIV.

One of the most preferable nucleotide sequences useful to include in the chimeric Expression Constructs of the invention are the so-called "ribozymes". These are useful in that once bound to a target nucleotide sequence, they can actually cleave the target sequence at a particular area, which ultimately enhances the destruction, reduction, or inhibition of the sequence, and where appropriate, its ability to express protein. One skilled in the art will understand that many so-called ribozyme sequences are known today, such as the "hairpin" as described in Ojwang. J.O., et. al., PNAS U.S.A., Nov. 15, 1992, Vol. 89, No. 22, pp. 108012-10806, and that described in Hampel, A. et al. Nucleic Acids Res. 18:299-304 (1990); RNase P, as described in McClain W.H. et al., Science 238:527-530 (1989), and Forster, A. and Altman, S., Science 244:78-786 (1990); Hepatitis Delta Virus Ribozyme, Thill, G., et. al. Nucleic Acids Res., Vol. 19, No. 23, 6519-6525, and Rosenstein, S.P. and Been, M.D., Nucleic Acids Res. 19:5409-5416 (1991); "hammerhead", Haseloff and Gerlach, infra; WO 92/01786; Tetrahymena as reported in Woodson, S.A. and Cech, T.R., Cell 57:335-345 (1989), Zaugh, A.J., et. al., Nature 324:429-433, and U.S. Patent Nos. 5,037,746 and 4,987,071; and the like, and could be inserted into the expression constructs to render chimeric constructs in accordance with the teachings set forth herein.

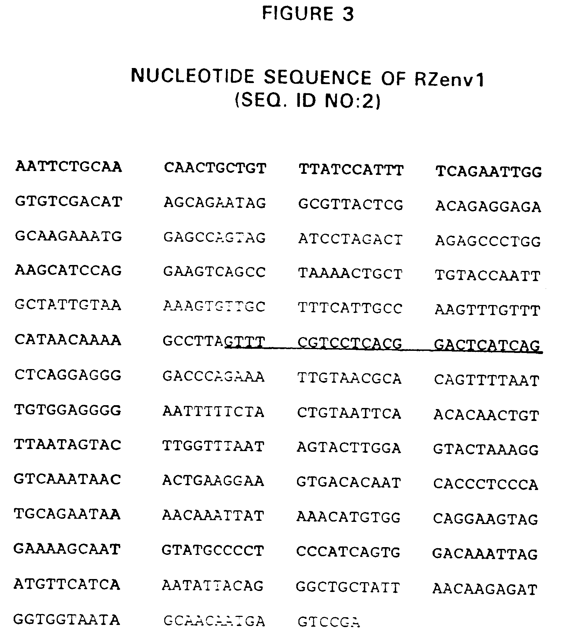

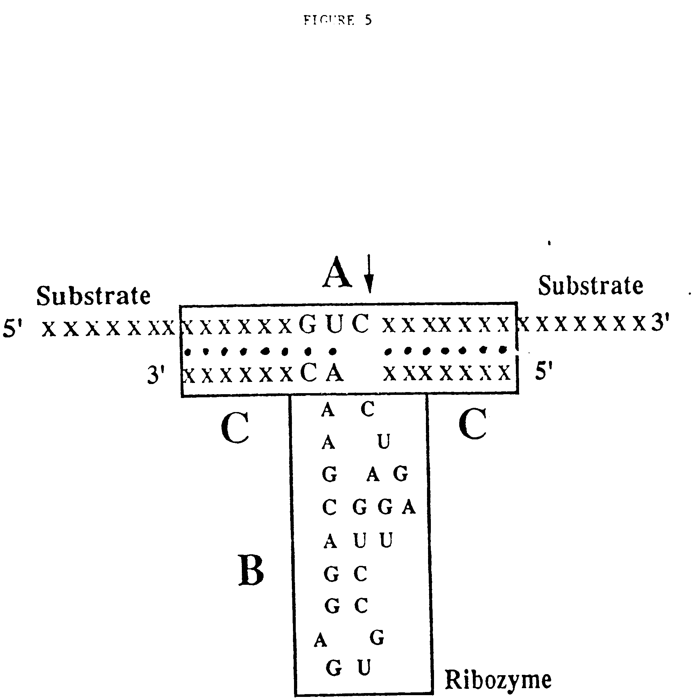

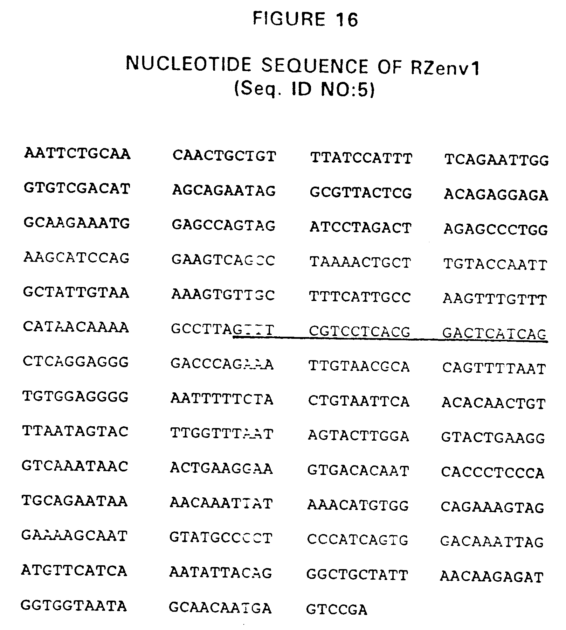

One of the earliest discovered and most notable ribozyme is the "hammerhead" sequence, discovered by Haseloff and Gerlach and reported in Nature 334: 585-591, 1988 and Gene 82:43-52 (1989). These ribozymes are preferred for use herein due to the fact that they are highly specific, with a 22 base catalytic sequence flanked by a 16 base substrate binding sequence (as schematically illustrated in Figure 5). The RNA of the ribozyme could bind to complementary RNA and cleave it immediately past the triplet GUC, GUA, GUU, CUC, AUC, or UUC. Placing the ribozyme amidst or adjacent to long stretches of antisense sequences as described above, facilitates its binding to target RNA, and successful cleavage. The preferred hybrid construct contains this ribozyme and antisense segments complementary to portions of the envelope region of HIV, due to the presence in these regions of the ribozyme recognition sequence, GUC, at one of two restriction enzyme sites. One such preferred hybrid has been prepared (its sequence is set forth in Figure 3, or a variant thereof which sequence is shown in Figure 16) wherein the underlined portions represent the nucleotide sequence for the ribozyme portion and has been designated "RZenv 1" (SEQ. ID NO:2, or depending on the strain, SEQ. ID NO:5).

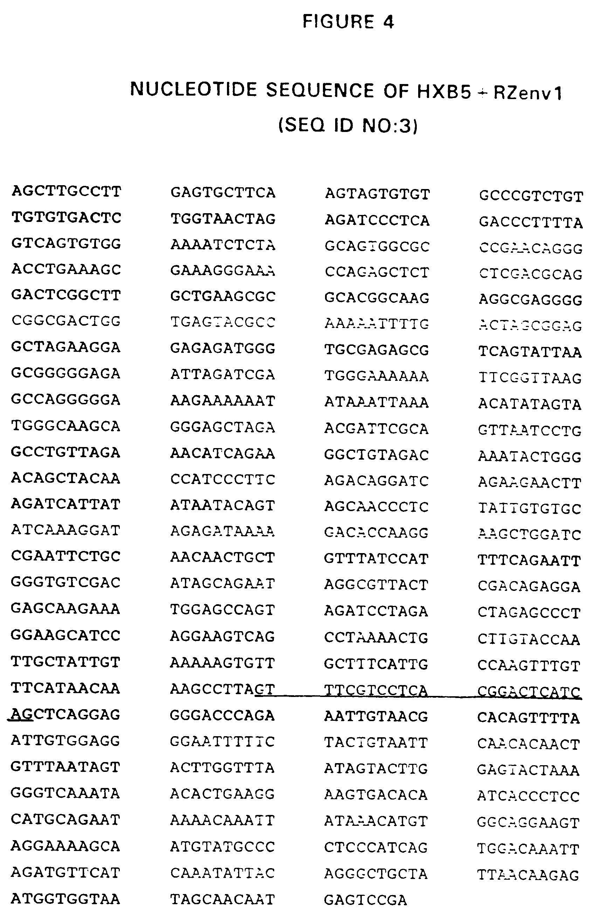

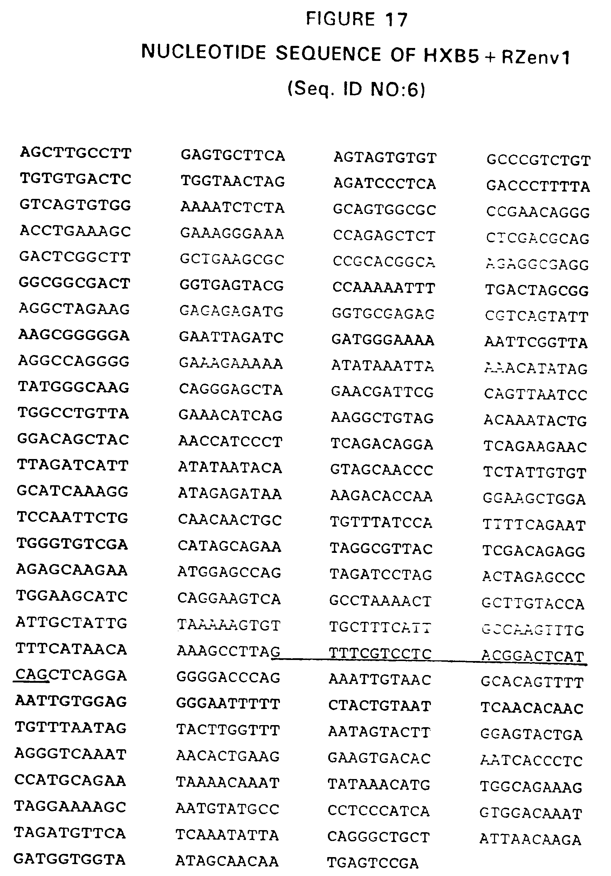

This construct contains a 500bp fragment of HIV env (Nts 5320-5534 and 6891-7197), which would be oriented antisense to the TAT exon2, including the start codon. This construct further contains a ribozyme against nt 6891 of the env region cloned in the sense orientation amidst the two env sequences, that are cloned in antisense orientation. A further preferred chimeric Expression Construct comprises HXB5 (SEQ. ID NO:1 or SEQ. ID NO: 4) tandem to RZenv 1 (SEQ. ID NO:2 or SEQ. ID NO:5). These constructs could be tandem to one another in either order, but in a preferred embodiment, HXB5 (SEQ. ID NO:1 or SEQ. ID NO:4) would be inserted in an expression vector downstream (3') of the RZenv1 sequence, as depicted in Figure 4 or in Figure 17, depending on the HIV strain (the ribozyme portions again underlined) (SEQ. ID NO: 3 or SEQ. ID NO: 6, depending on the strain).

Controls are also provided for these ribozyme containing constructs, with the ribozyme portion maintained in the antisense orientation. In this manner, one can judge the extent to which the ribozyme portion of the construct contributes to the overall inhibition of the virus, when compared to constructs that contain only antisense.

Subclones, mutations, and additional sequences such as cloning site sequences, flanking sequences, primer sequences, and the like relating to the Constructs described herein are also included within the scope of the invention, and may be included in the Expression Constructs of the invention. As long as the basic ability of the construct to bind to its target, and to then effect a substantial retardation or inhibition of the HIV virus is not greatly reduced, such changes are within the scope of the invention. Use of the Expression Constructs in combination is also within the contemplation of the invention. By "combination" is meant that the constructs are contacted with their target in a sequential or concurrent fashion in a delivery vehicle or some other delivery system, such as a retrovirus delivery system. In the latter case, the constructs could be present on the same retroviral expression vector, or on separate vectors, being delivered to the host cell or other target separately in tandem or concurrent fashion. A particularly preferred combination comprises the use of HXB5 (SEQ. ID NO:1 or SEQ. ID NO.:4) in conjunction with RZenv1 (SEQ. ID NO:2 or SEQ. ID NO: 5, depending on the strain) (SEQ. ID NO:3 or SEQ. ID NO:6, depending on the respective strains). The ribozyme sequence cleaves the area in the HIV genome that it binds to, while the other portions of the construct bind to various other regions of the HIV RNA, inhibiting its activities.

The efficacy of the Expression Constructs made in accordance with the present invention may be tested in a variety of assays known to the art. For example, HIV-1 p24 antigen expression is an accepted technique to mark the inhibition of HIV replication and expression. When HIV virus infects cells, and reproduces to produce more virion particles, p24 is detectable. Therefore, inhibition of p24 indicates that the virus replication process is inhibited, and "molecular immunity" is conferred. Certain preferred constructs of the invention demonstrated extended inhibition of p24 expression as further discussed in the Examples, and retardation of syncytia formation. Other useful assays that one may avail oneself of in testing the inhibitory abilities of the constructs made in accordance with the invention are assays that detect HIV RNA levels, reverse transcriptase assays, and the like.

The usefulness of certain of the Expression Constructs of the invention may be enhanced through chemical modification of the polynucleotide constructs to enhance their half-life. These include the following which are herein incorporated by reference: Terminal blocking group technique: Zamecknik, et al., Proc. Natl. Acad. Sci. 75, 28-284; Zamecknik et. al.,Proc. Natl. Aca. Sci. 83, 4143-4146; internucleoside methyphosphonates: Miller et. al., Biochimie 67, 769-776, 1985; Agris et. al., Biochemistry 25, 6268-6275, 1986; Smith et. al. Proc. natl. Aca. sci. 83, 2787-2791, 1986; phosphorothioates: Matsukura, et. al., Proc. Natl. Aca. Sci. 84, 7706-7710 (1987); covalent addition of polysine: Lemaitre et al., Proc. Natl. Aca. Sci. 84, 648-652 (1987); Lemaitre et. al., Nucleosides Nucleotides 6, 311-315 (1987); intercalating agents: Zerial et. al. Nucleic Acids Res. 15, 9909-9919 (1987); EP 138 437 to Genex (Enzon Labs, Inc.); Tung, et. al., Bioconjug Chem., Nov.-Dec. 1991, Vol. 2 (6), pp. 464465, and the like.

Delivery of the Expression Constructs to the target cells may be effected in conventional manner as will be known to those skilled in the art, and as described infra for the therapeutic composition embodiments of the invention. However, a preferred method of Expression Construct delivery is through the use of Retroviral Vectors, preferably in combination with packaging cells to produce Recombinant Retroviruses capable of targeting and infecting cells.

Retroviral transfer is a promising therapeutic technique due to its high and efficient level of expression, its ability to be produced in a large proportion of target cells, and its broad host range and capability for specific cellular targeting. Retroviral transfer can introduce the functional Expression Constructs of the invention into cells at one copy per cell, without affecting the proliferative capacity of the recipient cell. Additional details on available retrovirus vectors and their uses may be found in EPA 0 178 220, U.S. Patent No. 4,405,712, EPA 0 334 301, WO 89/11539, WO 91/10728, WO 92/07943 (especially useful for in vivo genetic manipulation of "patient cells" in that selective markers for detecting genetically modified cells are not needed), and the like. The teachings of the aforementioned publications are herein incorporated by reference.

The use of Retroviral Vectors, carrying nucleotide sequences of interest (whether or not in conjunction with a packaging cell line to fabricate a Recombinant Retrovirus) is better understood by reviewing the background and special properties of retroviruses. Retroviruses occurring in nature share certain defined characteristics. The genome of a retrovirus which is not replication defective is composed of an RNA molecule possessing, in the direction of the transcription (5'---3') an identical short sequence at each end, known as R (between 21 and 80 bases). This is followed, in order, by a single sequence known as U5 (from 70 to 80 bases), a tRNA binding site (PBS, approximately 20 bases), and a non-coding sequence (leader sequence). The RNA molecule continues with a region coding for three genes, the translation products of which are essential for the replication of the virus, and which are gag (virion structural proteins), pol (reverse polymerase) and env (envelope). The genome terminates, in order, with a non-coding sequence, a purinerich sequence (OPU), a single sequence known as U3 (from 200 to 450 bases), and finally the R sequence. The repeat end sequences (R) or single sequences (U5 and U3) peculiar to retroviruses appear to be rather well conserved in this group of viruses and contain the signals involved in the control of the expression of the viral genome.

The cycle of infection by a retrovirus begins with the adsorption of the virions on the surface of the cells, followed by penetration into the cytoplasm. In the cytoplasm of the cell, the single-stranded viral RNA is transcribed by the reverse polymerase present in the virion, to a linear copy of double-stranded DNA. The DNA copy resulting from this reverse transcription is slightly longer than the viral RNA molecule which acted as a template for it. This difference is the result of the addition of a U3 sequence at the 5' end and a U5 sequence at the 3' end. The combination, in order, of the U3-R-U5 sequences constitutes a repeat sequence at both ends of the DNA molecule, previously described as the LTR (Long Terminal Repeat). The copies of viral DNA containing one or two LTR sequences are conveyed to the nucleus where they are converted to molecules of circular shape. Some circular molecules only retain a single LTR. These molecules are then integrated in the cell genomic DNA. The viral DNA is integrated in the cell DNA in such a manner that it is enclosed by an LTR at each end, and then bears the name of proviral DNA or "provirus". The provirus acts as a template for the transcription of viral RNA molecules. The transcription is initiated at the R sequence of the left LTR and stops beyond the polyadenylation signal carried by the U3 or R sequence of the right LTR. The RNA molecules obtained after transcription of the provirus are a reflection of the mRNA of the eucaryotic cells, "capped" by a terminal 7mG residue at the 5' end, and are encapsidized in the viral protein envelope. The viral particles form an assembly which sediments at 70S. They are ejected outside the cell which produces them and proceed to infect the neighboring cells. This release of viral particles is not accompanied by the death of the cell.

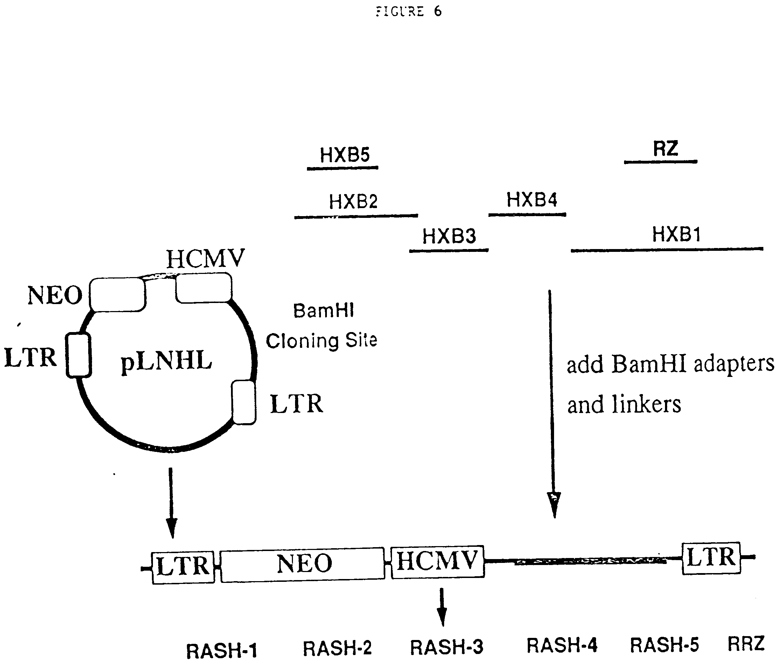

One skilled in the art will understand that there are a number of starting retroviruses to choose from to begin the construction of the recombinant Retroviral Vectors of the invention. Of these may be mentioned the avian retroviruses such as the avian erythroblastosis virus, avian sarcoma viruses, and the like, the murine sarcoma viruses such as murine Rous sarcoma, and leukemia viruses such as mouse Moloney leukemia virus. Certain starting retroviral vector plasmids may be obtained commercially, such as pXT1 (available from Stratagene, La Jolla, California). One such retroviral vector plasmid is depicted in Figure 6, as "pLNHL", wherein the viral protein (gag, pol, and env) coding sequences have been replaced with a dominant drug resistance marker gene and a promoter. The retroviral vector plasmid contains cloning sites appropriate for uses herein, the neomycin resistance gene (NEO), an internal promoter, human cytomegalovirus (HCMV), and murine leukemia virus (MLV) sequences flanking the 5' LTR and 3' LTR, which render the vector more convenient for cloning. The various Expression Constructs of the invention may be conveniently cloned into vector plasmids such as these.

The recombinant vectors as described herein may include other useful nucleotide sequences operationally associated with the Expression Construct, such as enhancer sequences. Enhancer sequences work to affect the ratio of transcription, and certain cell specific activities. Preferred enhancers for use with mammalian cells are obtained from mouse creatine kinase, enhancers from the T cell receptor, IL-2 enhancers, and the like.

Another element which may be present in the recombinant vector constructs of the invention is the polyadenylation site. Eucaryotic polyadenylation sites are well known, and hence, DNA sequences can be obtained in accord with published reports. Exemplary polyadenylation sequences can be obtained from mouse beta-globulin, and simian virus 40 late or early region genes, but viral polyadenylation sites are preferred. The sequences are known and may be ligated to the vectors in conventional fashion.

A suitable selection marker may be included in the recombinant vector construct. Various marker gene sequences are known to the art and can be chosen for purposes herein. However, the marker which will be expressed in the test cells or in secondary test cells, is preferably a gene sequence encoding a protein selected from i). an enzyme; (ii) a protein which will enable the test cells or secondary test cells to grow in a medium in which they would not otherwise grow, such as in the presence of an otherwise toxic drug, or in the presence of a selective media deficient in certain key factors required for cell growth, and the like; and (iii) an antigen, and, in particular, one which will be expressed on the cell surface. Examples include aminoglycoside-phosphotransferase (APH) selected for by the inhibition of G418, Dihydrofolate reductase (DHFR): Methotrexate resistant variant, Hygromycin-B-phosphotransferase (HPH), selected for by the inhibition of Hygromycin-B which would ordinarily inhibit protein synthesis, Thymidine kinase (TK), selected for through the use of drug Aminopterin which would ordinarily inhibit de novo purine and thymidylate synthesis (TK synthesizes thymidylate), Xanthine-guanine phosphoribosyltransferase (XGPRT), selected for through the use of Mycophenolic acid which ordinarily inhibits de novo GMP synthesis (XGPRT synthesizes GMP from xanthine), and Adenosine deaminase (ADA) which inactivates the addition of a compound that damages DNA. Preferred for use herein are the dominant selection markers, such as such as neomycin, GPT, and HGPRT, as you do not need a mutant cell line for expression.

Suitable promoters include those that function in a variety of cells. Examples of these are SV40, RSV, murine leukemia virus, phophotidykinase (PGK), HCMV, TK promotor and the like. Preferred for use herein is HCMV because it is a strong promotor capable of operation in a variety of cell types. It may also be useful to include promoters that are externally regulatable.

The Retroviral Vectors of the invention may include other constructs of interest. For example, in a particularly preferred embodiment, a nucleotide sequence expressing soluble CD4 antigen may be included in the Retroviral Vector. The CD4 molecule is one of several non-polymorphic T lymphocyte surface proteins that have been implicated in the mediation of efficient T cell-target cell interactions. Soluble CD4 protein has been characterized in terms of its biological and immunological properties, and has been considered of value in the prevention and treatment of HIV infection. Studies indicate that the protein may act as an inhibitor of extracellular and cell to cell spread of the virus. Because soluble CD4 has been shown to block virus binding to CD4 positive target cells in culture, it is believed that administration of the protein to infected persons would act to inhibit the extracellular spread of the virus. It is therefore of value both in a prophylactic sense and as a therapeutic agent in the treatment of the disease state in infected persons. As a prophylactic, CD4 may be administered to individuals who show exposure to HIV by the presence of antibodies to the virus. Administration of an effective amount of the protein at an early stage of the disease or prior to its onset acts to inhibit infection of CD4 positive lymphocytes by HIV. As a therapeutic, administration of the protein to persons infected with the HIV acts to inhibit extracellular spread of the virus. Fusion between HIV infected cells and other CD4 positive lymphocytes also appears to be a route of virus spread. Fusion may also be responsible for the impairment of CD4 lymphocyte function and ultimately the depletion of the CD4 positive lymphocytes in infected individuals. Cell fusion is dependent on both viral envelope gene products and the CD4 receptor. Soluble CD4 interferes with cell fusion and therefore is expected to diminish the cell to cell spread of the virus and the loss of T4 lymphocyte function.

Thus, in certain combination embodiments of the invention, the Retroviral Vector is delivered to the target cell, wherein the antisense portion of the construct can operate to inhibit the viral infection, while soluble CD4 is advantageously produced in a relatively constituitive manner by the cell and secreted into the bloodstream to bind to virus (the gp120 on the virus particles) that is free in the bloodstream. This is a two-pronged attack on the HIV viral infection. In accordance with the present invention, various chimeric Expression Constructs are provided in the Retroviral Vectors of the invention, in combination with soluble CD4 or smaller functional subsets thereof. A plasmid comprising a cDNA sequence for sCD4 is designated pT4B and may be obtained from the American Type Culture Collection (ATCC) in Rockville, Maryland. Additional information on soluble CD4 is provided in EP 0 313 377 and U.S. Patent # 5,126,433 (Maddon et. al.), herein incorporated by reference. The location of such an insert, as more fully explained in the Examples may be cloned between the LTRs of the starting vector. Sometimes LTRs become nonfunctional, therefore it may be desirable to position the antisense next to the internal promoter. The selection marker may be positioned downstream of the 5' LTR or at any other convenient cloning site. When additional constructs are added, their location on the vector is preferably close to the promotor before the start codon between the transcription start site in the promoter and before ATG. In the particular case of the soluble CD4 gene, the gene, or fragment thereof, is preferably inserted between the U3 and U5 of the 3'LTR. This engineered LTR is then substituted for the 3' LTR of the starting retroviral vector. This avoids inserting CD4 downstream of the antisense and various interspersed start codons.

Preferred Retroviral Vectors of the invention include one or more of the aforementioned Expression Constructs, inserted into a retroviral vector (Figure 6), and particularly HBX4 and pRRZ env 1, which complete Retroviral Vector embodiments are hereinafter referred to as "RASH4" and "RRZ env". Also preferred for use herein are recombinant Retroviral Vectors containing a combination of antisense Expression Constructs, alone or in combination with another construct of interest. Particularly preferred in this regard is a Retroviral Vector comprising HXB5 (SEQ. ID NO:1 or SEQ. ID NO:4), RZenv1 (SEQ. ID NO:2 or SEQ. ID NO:5), and soluble CD4 (RASH8). In these preferred embodiments, promoters of choice include CMV, SV40, RSV, and pGK, and preferred selection markers are Neo, GPT, and HGPRT.

The Retroviral Vectors of the invention may be administered therapeutically (in vivo or ex vivo) by direct administration in a suitable medium, as described below in detail for the therapeutic composition embodiments, or they may be contained in a delivery system such as liposomes, or viruses such as baculovirus, adenovirus, vaccinia virus, unrelated retroviruses, and the like. These viruses are known to express relatively high levels of proteins from exogenous genes provided therein. For DNA virus vectors, recombinant DNA viruses can be produced by in vivo recombination in tissue culture between viral DNA and plasmids carrying retroviral or retroviral vector genetic information. The resultant DNA viral vectors carrying either sequences coding for retroviral proteins or for retroviral vector RNA may be purified into high titre stocks. Alternatively, the constructs can be constructed in vitro and subsequently transfected into cells which provide in trans, viral functions missing from the DNA vectors. Susceptible cells may then be infected, with the result that expression of the retroviral proteins or RNA retroviral vector genomes, or both are suitably expressed. This technique, when used with adenovirus or other mammalian vectors, allows the use of cells typically used for expression, to produce recombinant retroviral vectors. See WO 91/02805 for more information in this regard, herein incorporated by reference.

In embodiments using strictly retrovirus systems, the Retroviral Vectors, if deficient in packaging protein-encoding sequences, may also be "rescued" as packaged retrovirions from the targeted cells, by introducing into a construct transfected cell (either concurrently or sequentially), a "helper retroviral vector". These helper viral vectors express packaging proteins within the cells that are necessary for viral replication. The helper virus is often rendered defective by removing its own packaging sequences, so that it produces the needed proteins to package the construct-encoded RNA genome, but does not package its own genome. Such helper virus would be selected so as to avoid adverse effects if used in therapeutic administration.

The Retroviral Vectors of the invention may also be packaged into the so-called "packaging cells" to produce a complete functional Recombinant Retrovirus that will infect cells as its counterparts in nature do. Packaging cell lines may be obtained commercially, from the American Type Culture Collection (ATCC), or fabricated in accordance with a variety of teachings. Mulligan and Danos have described one such useful cell line in WO 90/02806. These and related cell lines are available from MIT, Cambridge, Massachusetts. Other useful cell lines and teachings include those described in WO 90/02797, from North Carolina State University, and WO 89/11539, Gilboa from Sloan-Kettering Institute for Cancer Research, those described in WO 89/07150, Arthur Bank from Columbia University, and those described in WO 86/00922, Inder Verma from the Salk Institute for Biological Studies. The teachings set forth in the aforementioned publications are herein incorporated by reference.

The preferred delivery of the Retroviral Vectors of the invention is through the use of the aforementioned Recombinant Retrovirus. The preferred Recombinant Retrovirus genomes of the present invention are "replication defective", which, as described herein means that they are incapable of reproducing viral genome encapsidated viral particles in cells (functional virions) in the cells they infect (they could, however, produce empty virions that bud from the cell). Furthermore, they do not yield substantial helper virus, nor do they substantially transfer the viral packaging function to progeny virus. However, they do replicate in the host cellular genome during that cell's normal cellular mitosis activities. Elements required for retrovirus replication can be divided into cis- and trans-acting factors. The transacting factors include proteins encoded by the viral genome that are required for encapsidation of the viral RNA, entry of virions into cells, reverse transcription of the viral genome, and integration of the DNA form of the virus into host DNA. The cis-acting factors include signals present in the viral RNA which interact with these proteins and other factors during virus replication. U.S. Patent #4,980,289 describes a recombinant retrovirus which is replication deficient rendered through a deficiency in the retrovirus promoter sequence. U.S. Patent #4,861,719 describes a retroviral packaging cell line that has a low propensity for generation of replication competent viruses in that the genome of a replication competent virus is systematically altered to maximally interfere with the cis-acting elements while preserving production of the trans-acting elements. The cell lines described therein do not transmit the virus, but will transmit other RNAs containing the proper cis-acting elements, including retroviral vectors designed to carry foreign genes. One of the more recent approaches to constructing safer packaging cell lines involves the use of complementary portions of helper virus, divided among two separate plasmids, one containing gag and pol, and the other containing env, Markowitz et al., Virology 167: 600-606, 1988. A benefit of this system is that three recombination events are required to generate a replication competent genome. Mulligan and Danos describe this and other useful cell lines, based upon sequential transfection techniques (rather than concurrent transfection) WO 90/02806, as does Banks WO 89/07150, herein incorporated by reference. Preferred for use herein are the packaging cell lines as described in Banks, because wild-type helper-free Producer Cells can be made using these. Generally, the packaging sequence and the 3' LTR are also deleted in each of the helper plasmids in the Banks' cell lines. The resulting recombinant retroviruses are amphotropic, capable of targeting a wide variety of cellular types to deliver the foreign genetic material of interest.

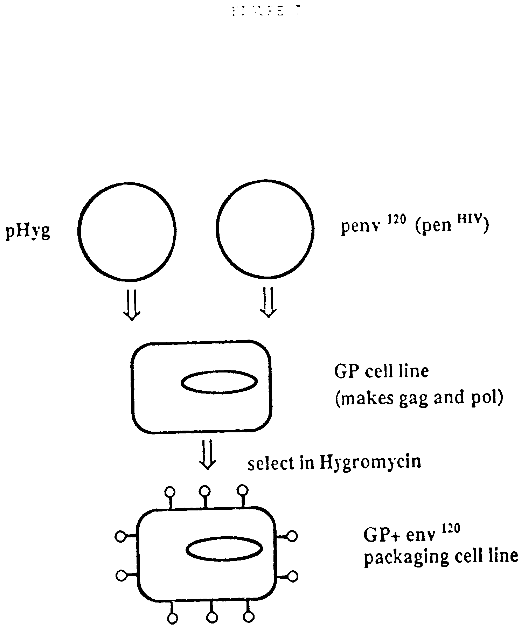

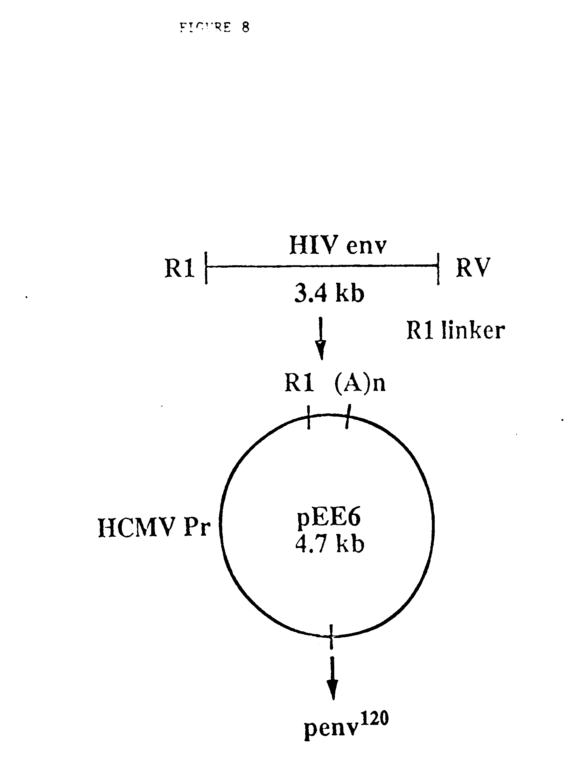

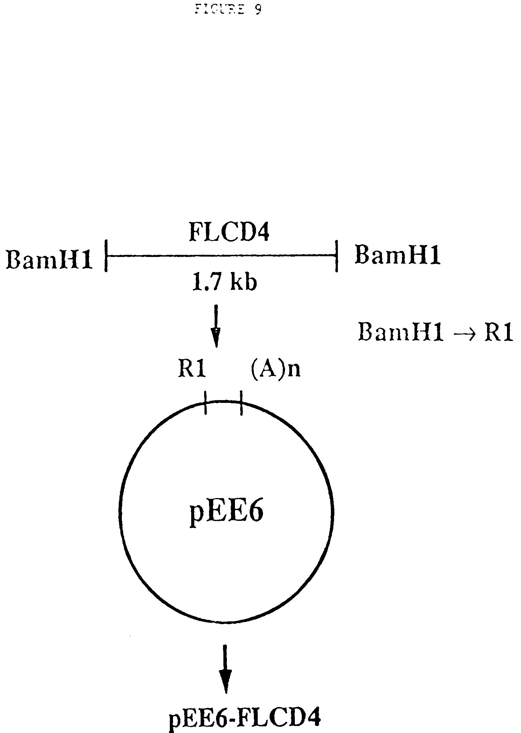

In a further aspect of the present invention, there is provided a Recombinant Retrovirus capable of infecting cells bearing certain surface ligands (or receptors), and delivering the Retroviral Vector to such cells. In these embodiments, the Retroviral Vector comprises the Expression Constructs of the invention incorporated into suitable packaging cell lines that carry additional genetic inserts that are capable of expressing a particular ligand on the envelope of the Recombinant Retrovirus. The constructed virus displays a number of surface ligands that will bind to receptors on select target cells, thereby gaining entry to such cells, to the exclusion of other cells that do not express that particular receptor. In one embodiment, erythropoietin is the ligand expressed, yielding a Recombinant Retrovirus capable of delivering vector material to cells expressing the "EPO" receptor, such as erythroid precursor cells. In certain other preferred embodiments, the CD4 receptor is expressed on the surface of the Recombinant Retrovirus particles, and targets T lymphocytes or monocytes that display the HIV gp120 protein (in other words, cells infected with HIV). The Recombinant Retrovirus so constructed is able to gain entry to the cell of interest, in a highly selective manner. Cells not bearing the gp120 protein will not be infected. In a further preferred embodiment, the Recombinant Retrovirus displays gp120 on its surface, and targets CD4 cells. The host cell range specificity of a retrovirus is determined in part by the env gene products. Coffin, J. (RNA Tumor Viruses 2:25-27, Cold Spring Harbor, 1985) notes that the extracellular component of the proteins from murine leukemia virus (MLV) and Rous Sarcoma virus (RSV) are responsible for specific receptor binding. The cytoplasmic domain of envelope proteins, on the other hand, are understood to play a role in virion formation. Accordingly, a hybrid env product may be created comprising an env protein having cytoplasmic regions and exogenous binding regions which are not in the same protein molecule in nature. In this manner, a Recombinant Retrovirus is created with "tailor-made" specificity for target cells. The Examples set forth this process in greater detail. Figure 7 schematically depicts the construction of a preferred CD4 tropic packaging cell line of the invention, which becomes a Producer Cell for the Recombinant Retrovirus. The cell line may be prepared by transfecting a vector encoding for env and the ligand of interest (in this case gp120 or CD4) into a cell line that is capable of encoding gag and pol. The vector containing the env may be a non-retroviral vector, such as pEE6 commercially available from Celltech, England (Figures 8 and 9).

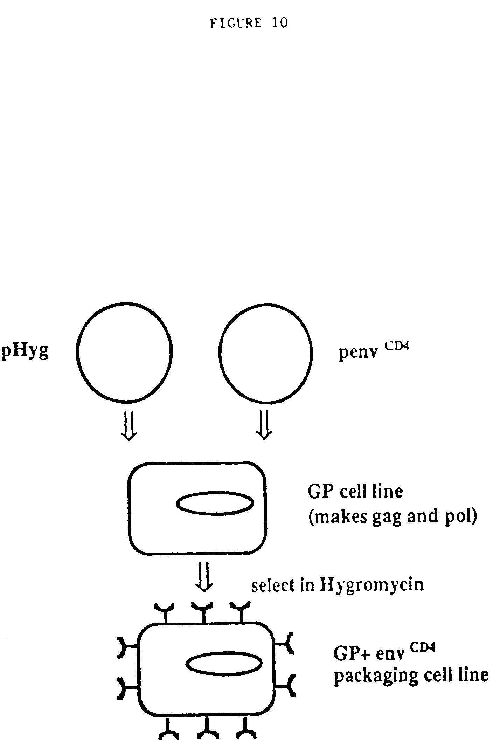

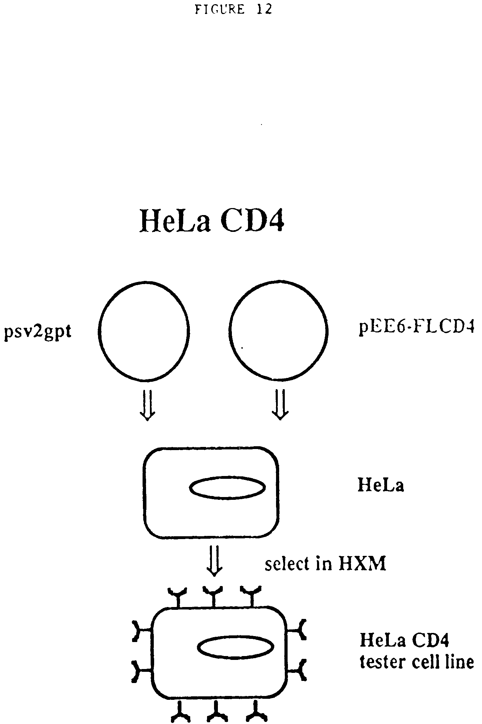

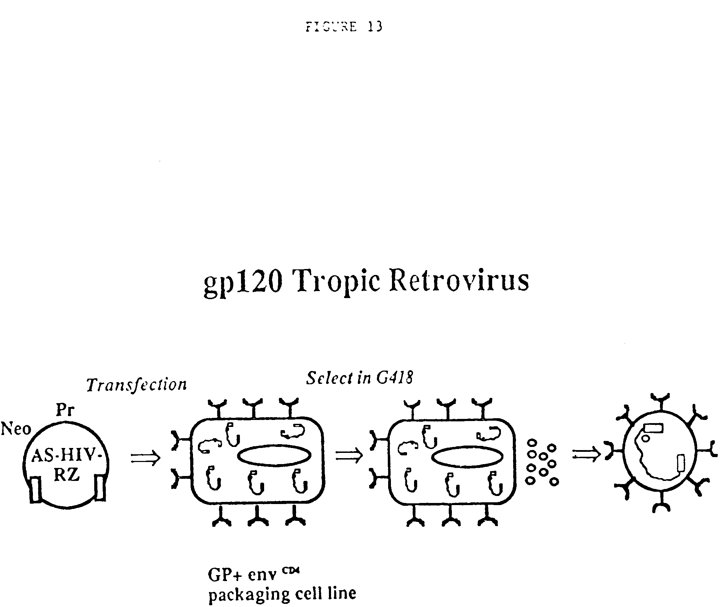

The retroviral structural proteins are provided separately to render the recombinant virus replication incompetent, in accordance with the teachings herein. The gag and pol viral components are provided through the use of a second vector, containing such components, such as may be obtained from cell lines containing these components from Mulligan, (MIT) or Arthur Banks from Columbia University. Plasmids carrying portions of the HIV genome may be obtained from Dr.Montagnier, Institute Pasteur, France, and the plasmid pHXB2B may be obtained from Gallo. Selective markers may be included on one of the two vectors or they may be provided on a separate vector and co-transfected (Figures 7 and 10). Retroviral Vectors of the invention are transfected into such targeting packaging cell lines, and thereby packaged (Figure 13).

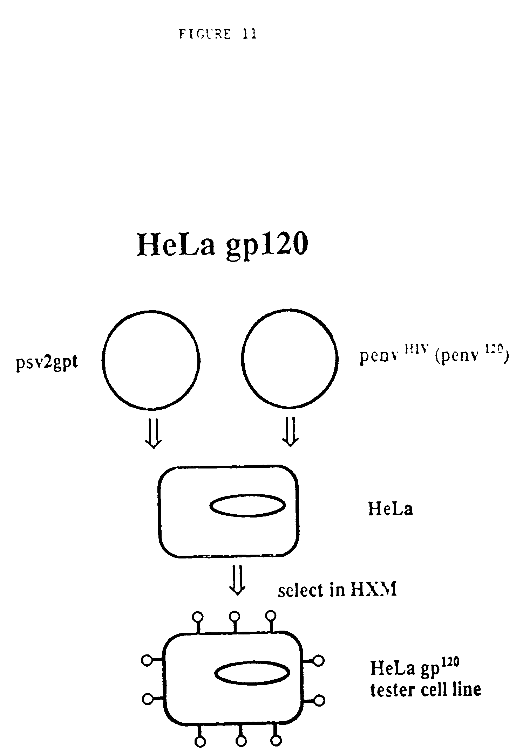

One skilled in the art will understand that in constructing such packaging cell lines, their ability to target the appropriate cells may be tested outside the patient, through the use of cell lines that express the receptor. In the case of gp120, CD4+ cells, such as CEM, HeLA CD4+, and the like may be used (Figure 12). In the case of CD4, HIV-infected cells, or a HeLa gp120 tester cell line (Figure 12) may be used. The preferred packaging cells of the invention have demonstrated superior targeting properties.

In yet a further aspect, the invention concerns a cell which has been transformed with the expression vectors of the invention, with the result that resistance to retroviral infection of said cell is conferred. The transduced cell may be any of a number of cell types, including stem cells, leukocytes, erythroid precursors, and the like, and comprises one or more of the Expression Constructs of the invention, which directs transcription of RNA within said cell, said RNA containing at least in part, a polynucleotide sequence which is substantially complementary or homologous to one or more regions within the viral genomic RNA or mRNA transcript of the genomic RNA, and is effective to substantially inhibit replication of said retrovirus.

In accordance with another aspect of the present invention, there is provided a method of introducing foreign nucleic acid into a cell by creating a vector comprising an Expression Construct of the invention, and infecting the cell with such vector. The cells may be part of a living organism.

The invention also concerns a progeny of said cell so transformed, said progeny containing a sequence which is descendant from said Expression Construct sequences of the invention, and is also effective to inhibit replication of said retrovirus, with the result that molecular immunity has been conferred upon the cells.

Further embodiments of the invention include therapeutic compositions including the Expression Constructs, the Retroviral Vectors, or the Recombinant Retroviruses in conjunction with any medium for storage, and capable of being rendered compatible for therapeutic administration, in vivo or ex vivo.

The therapeutic compositions of the invention may be lyophilized or provided in solution or suspension, and stored frozen or at room temperature. Suitable suspension media include physiologically compatible media, such as phosphate buffered saline, Dulbeccos Modified Eagle's Medium (DMEM), MEM, Gibco's medium, pyrogen-free distilled water, and the like.

The mode of administration of the preparations of the invention may determine the sites and/or cells in the organism to which the construct(s) will be delivered. The compositions of the invention can be administered alone but will generally be administered in admixture with a pharmaceutical carrier or diluent selected with regard to the intended route of administration and standard pharmaceutical practice. The constructs may be administered directly to the patient by any suitable mode, such as parenteral, subcutaneous, intramuscular, intraperitoneal, intradermal, intra-arterial, and the like, by use of a catheter or other routine administration routes. In the case of direct administration, one has to rely upon the ability of the Expression Construct to efficiently target the appropriate cells or other endpoints it is desired to target. Generally, the Expression Constructs will be utilized in purified form together with pharmacologically appropriate carriers. Typically these carriers include aqueous or alcoholic/aqueous solutions, emulsions, or suspensions, including saline and buffered media. Parenteral vehicles are delivered in the form of sterile solutions, and include sodium chloride solution, Ringer's dextrose, dextrose and sodium chloride solution, and lactated Ringer's, and may contain other solutes, for example, enough salts or glucose to make the solution isotonic. Suitable physiologically acceptable adjuvants, if necessary to keep the complex in suspension, may be chosen from thickeners such as carboxymethylcellulose, polyvinylpyrrolidone, gelatin, and alginates. Intravenous vehicles include fluid and nutrient replenishers and electrolyte replenishers, such as those based on Ringer's dextrose. Preservatives and other additives, such as antimicrobials, antioxidants, chelating agents and inert gases, can also be present.

Alternatively, the therapeutic compositions may be administered to cells ex vivo, through a variety of routes, and then the transformed cells are transplanted into the patient by graft or otherwise. Certain grafting techniques using genetically treated cells are exemplified in U.S. patent #4,980,286, herein incorporated by reference. Specifically within the contemplation of the invention is the treatment of blood through phoresis techniques. Other traditional techniques and transfection protocols for introducing nucleic acid sequences into cells are useful for transferring the Expression Constructs to the target of interest. These techniques include calcium-phosphate mediated techniques, DEAE-dextran mediated transfection techniques, lysozyme fusion or erythrocyte fusion, osmotic or sucrose shock, scraping, or direct uptake by the cells from their surroundings. These techniques may be augmented by subjecting the cells to electric currents. The constructs may also be microinjected directly into their target area using the so-called "gene gun".

Delivery systems such as liposomes have also been reported as useful for delivery of genetic information to a target of interest (WO 91/02805). See generally, U.S. patent 4,880,635 to Janoff et al. which provides a useful description of techniques for dehydrated liposomes, and cites a great deal of the art in the general area of using liposome preparations for drug delivery.

One skilled in the art will also understand that certain of these last-mentioned techniques may actually be useful for in vivo administration of the constructs. For example, microinjection might be quite satisfactory for delivering the engineered materials of the invention to a patient's bloodstream, or some other more readily accessible portion of the body.

In certain clinical use embodiments, patients may be innoculated directly with the vectors of the invention and could have their T cells, not yet harboring the HIV virus, infected with the Expression Constructs, resulting in Stably Transformed Cells that express anti-sense polynucleotides and other nucleotides of interest. These therapeutically transformed cells would than be resistant to infection by the pathogenic retrovirus. This strategy would stop or retard the progression of the disease, and the resistant T-cells or other stem cells might serve to reconstitute the patient's immune system. Alternatively, precursors to T-cells, such as marrow stem cells, fetal thymus, and the like may be explanted from the patient or donor, infected at high multiplicity by viral stocks of the Retroviral Vectors of the invention and transplanted into the patient using available transplantation technology. Cells successfully infected with the vectors would reconstitute the patient's immune system with T-cells resistant to infection by residual pathogenic virus.

For administration to humans in the treatment of disease states responding to such therapy, the prescribing physician will ultimately determine the appropriate dosage for a given human subject, and this can be expected to vary according to the weight, age and response of the individual as well as the nature and severity of the patient's disease.

### MATERIALS AND METHODS

### CONSTRUCTION OF ANTISENSE HIV CONTAINING RETROVIRAL CONSTRUCTS

### pRASH-1, pRASH-2, pRASH-3, pRASH-4

The source of the antisense HIV sequences is the cloned HIV proviral DNA contained in SP46, the construct being designated pHXB2D and obtained from Dr. Robert Gallo of NIH. pHXB2D was cut with Eco R1 + EcoRV to obtain 4 large HIV genomic fragments:
1. HXb1 a 3.4 kb fragment spanning from nts 5322-8694 and containing env, tat, rev, U3 and R.
2. HXB2 a 2.8 klb fragment covering nts -342-2556 and containing R, U5, gag and 5' part of pol.
3. HXB3 a 1.7 kb fragment containing (nt 2556-4227) the middle party of pol.
4. HXB4 a 1.1 kb (nt 4227-5322) fragment containing the 3' part of pol + nef.

The ends of these fragments were changed to BamH1 ends and cloned into the BamH site of the Retroviral Vector pLNHL. It is to be mentioned that during the conversion to a BamH1 ends, HXB1 was reduced to 2.8 kb because of an internal BmH1 site (nt 8051). The resulting retroviral constructs are termed pRASH-1, pRASH-2, pRASH-3 and pRASH-4, respectively. (R= retrovirus, AS=antisense, H=HIV). The antisense orientations of the fragments were determined by ScaI (pRASH-1), Sph1 (pRASH-2), XmnI (pRASH-3) and BstEII + DraI (pRASH-4).

### pRASH-5

pRASH-2 was cut with HindIII+PstI to release a 550 bp fragment of HIV (nts 77-628), covering the R, U5, PDS, SD, Ψ and 5' gag portions. This is an important region in the life cycle of HIV. The HindIII ends of this fragment were converted to BamHI ends and cloned into pLNHL. The resulting construct is named pRASH5.

The antisense orientation was confirmed with SstI. (PstI was used in addition to HindIII to separate this fragment from same sized neighboring fragment (nts 628-1255) which has an internal PstI site (nt 958).

### pRASH7 Retroviral Construct

pRASH7: pRASH1 was cut with BamHI+BgIII and a 1.3 kb fragment (nts 5322-6617) containing tat, rev and 5' part of env and their splice signals isolated. This was then ligated to BamHI cut, phosphatased and purified pLNHL. The antisense orientation of the insert was confirmed with SstI.

pRASH8 Retroviral Construct

pRASH8: pRASH2 was cut with BgIII and a 1.6 kb fragment (nts 19-1639) containing the R, U5 and gag was isolated. This was liagted to BamHI cut, phosphatased and purified pRASH7. The antisense orientation of the insert was confirmed with SstI. So, this construct contains 2.9 kb of HIV sequences in the antisense orientation.

pRRZenv (this construct contains a ribozyme cloned amidst envelope sequence).

pRASHI was cut with BamHI to release the 2.8 kb HIV env sequence. It was subcloned into pBR322 to give pBRHXB1. This construct was treated with B5u36I (MstII) to cut away 1.3 kb fragment from the envelope sequence. A ribozyme was directionally cloned in the sense orientation into the Bsu36I site of the vector pBRHXBIΔ to give pBRHXBIΔRZ. This was cut with BamHi + Bg1II to release the 550 bp BmHI + Bg1II ended fragment, containing the ribozyme, This fragment was cloned back into the BamHI site of pLNHL to yield pRRZenv1. The antisense orientation of the envelope sequences was confirmed with ScaI.

pRRZenv1R Identical to pRRZenv except that the ribozyme is in the reverse (antisense) orientation.

pRRZenv1 - ASH5: pRASH5 was cut with BamH1 to give the BamH1 ended 550 bp HIV fragment. This was cloned into the BamH1 site of pRRZenv1. The antisense orientation of the 550 bp fragment was confirmed with SstI. This fragment is downstream (3') of the antisense RZenv insert in this construct.

pRRZenv1R-A5H5: the 550 bp fragment mentioned above was similarly cloned into pRRZenv1R.

The above-mentioned RASH and RRZenv constructs, including the controls, were separately transiently transfected into ecotropic packaging cell lines. The medium from these was harvested and used to infect amphotropic cell lines. Part of the infected cell lines were frozen, and part of them were put into G418 selection. Colonies developed in about two weeks. The media from these cells, which contains the respective viruses, is used to infect HIV target cells, such as CEM-T4 cells, which express high levels of CD4.

pRSCD4: A 1.2 kb SCD4 sequence was cut out of a pEE6-SCD4 construct with EcoRI. The ends were changed to BAmH1 and cloned into the BamH1 site of pLNHL.

p3'LTR/NheI-SCD4: The construct pRSCD4 was cut with XbaI + NheI to isolate 600 bp SCD4 sequence that is sufficient to code for a functional SCD4. This was cloned into the NheI site in the MLV 3'LTR contained in p3'LTR. The sense orientation was confirmed with BamH1 + EcoRI. It is to be noted that there is a stop codon immediately following the NheI site in frame with SCD4 sequence, so there was no need to add a stop codon to the 3' end of SCD4 fragment.

3'LTR/XbaI-SCD4: The XbaI + NheI ended 600 bp SCD4 fragment from pRSCD4 was cloned into the Xba1I site in the MLV 3'LTR in p3'LTR. Again, the sense orientation was confirmed with BamH1 + EcoRI. In this construct, the SCD4 coding sequence is extended by 14 amino acids coded by sequences in the LTR before termination.

pRASH5-SCD4: The retroviral construct pRASH5 was cut with C1aI and the large fragment containing MLV 5'LTR, Neo, HCMV promoter and ASHIV5 was isolated. This was then ligated to C1aI cut p3'LTR/NheI-SCD4.

pRRZ env1 - SCD4: The retroviral construct pRRZ env1 was cut with C1aI and the large fragment containing MLV5'LTR, Neo, HCMV promoter and RZenv1 (SEQ. ID NO:5) was isolated. This was ligated to C1aI cut p3'LTR/Nhe1 - SCD4.

pRRZ1 env1R-SCD4: The C1aI ended fragment containing MLV5'LTR, Neo, HCMV promoter and RZenv1R was ligated to C1aI cut 3'LTR/NheI-SCD4.

pRRZENV1 ASH5 - SCD4: The retroviral construct pRRZenv1 ASH5 was cleaved with C1aI. The large fragment containing MLV 5'LTR, Neo HCMV Promoter and RZenv1 (SEQ. ID NO:2) ASH 5 was ligated to C1aI cut p3'LTR/NheI - SCD4.

pRRZenv1R ASH5 - SCD4: The C1aI ended fragment containing MLV5'LTR, Neo, HCMV Promoter and RZenv1RASH5 was ligated to C1aI cut p3'LTR/NheI - SCD4.

### CONSTRUCTION OF NON-RETROVIRAL CONSTRUCTS:

penv¹²⁰: pHXB2D was cut with EcoRI+EcoRV and the 3.4 kb fragment encompassing the HIV envelope gene (gp160), tat and rev and the splice signals was isolated. The ends were converted to EcoRI ends and cloned into the EcoRI site of the eukaryotic expression vector pEE6, downstream of the HCMV promoter.

penvHIV: The EcoRI ended envelope fragment mentioned above was cloned into an EcoRI cut MLV vector downstream of the 5'LTR. This construct lacks the 3'LTR and the packaging signal, but has the splice donor site.

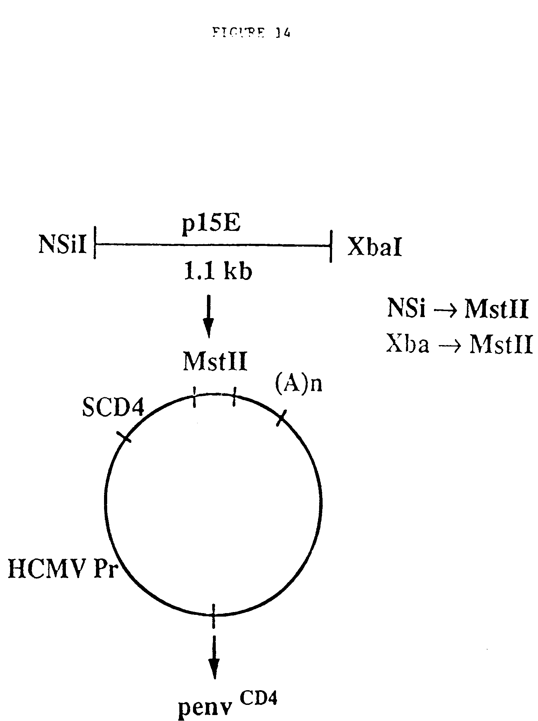

penv^{CD4}: The retroviral vector pLNHL was cut with NsiI + XbaI and the 1.1 kb MLV envelope region containing 3' part of gp70, and the transmembrane and cytoplasmic tail were isolated. The ends were converted to Bsu36I (MstII). This was inserted, in frame, into the unique Bsu36I site in the 3' portion of the SCD4 gene contained in the construct pEE6-SCD4. The MLV transmembrane cytoplasmic sequence of the HIV env will anchor the SCD4 to the membrane of the Recombinant Retrovirus. Figure 14 demonstrates this schematically.

penv^{EPO}: The NsiI + XbaI ends of the 1.1 kb MLV fragment mentioned under pen^{CD4} were changed to SphI ends. This was then inserted in frame, into the unique SphI site at the end of the human EPO gene sequence contained in pEPO, to yield pEPO-15E. the EPO-15E gene was cut out of pEPO-15E with ApaI + BamHi. The ends of EPO-15E were changed to EcoRI ends and the fragment was cloned into the EcoR1 site of pEE6 downstream of the HCMV promoter.

### ADDITIONAL RETROVIRAL CONSTRUCTS:

p3'LTR SV SCD4: The construct pSV2gpt is cut with PvuII + HindIII to isolate the 323 bp SV40 origin of replication (ori). The ends are converted to BamH1 ends and cloned into the BamH1 site of p3'LTR/nhel - SCD4 upstream of the SCD4 sequence. The correct orientation of ori is confirmed with AvrII + NheI.

pRASH5 - SV - SCD4: The retroviral construct pRASH5 is cut with C1aI and the large fragment containing MLV5'LTR, Neo, HCMV Promoter and ASH5 is isolated. This is ligated to C1aI cut p3'LTR SV SCD4.

pRRZENV1 - SV - SCDR: p3'LTR SV SCDR is cut with C1aI and ligated to the C1aI ended large fragment derived from pRRZENV1 and containing MLV5'LTR, Neo, HCMV promoter and RRZenv1.

pRRZENV1 ASH-SV-SCD4: The C1aI cut p3'LTR SV SCDR is ligated to the C1aI ended large fragment derived from pRRZENV1 ASH5 and containing MLV 5' LTR, Neo, HCMV Promoter, RRZENV1 and ASH5.

pRRZENV1RASH5 - SV-SCD4: The C1aI cut p3'LTR SV SCD4 is ligated to the C1aI ended large fragment containing MLV5'LTR, Neo, HCMV Promoter, RRZenv1R and ASH5.

### TESTING OF RETROVIRAL CONSTRUCTS (PROOF OF PRINCIPLE)

For the purpose of demonstrating the efficacy of the technology, PLNHL, pRASH-3 and pRASH-4 were chosen. They were separately transfected into the amphotropic packaging cell line GP+ENV^{am} and virus containing medium from G418 resistant colonies were used to infect the human T lymphoblastoid cell line (which is susceptible to HIV infection). G418 resistant cells were isolated and those infected with RASH3 and RASH4 were shown to express respective antisense RNAs. The cells were then challenged with HIV. RASH4 gave 80% protection against HIV whereas RASH3 gave 75% protection, as shown by p24 ELISA's and RT assays at 7 days post HIV infection.

### A COMPREHENSIVE RETROVIRAL CONSTRUCT

This would contain ASH6 RZenv1 (SEQ. ID NO:2 or SEQ. ID NO:5, depending on variant used), ASH6 (500 bp 3' pol fragment derived from PRASH-4) and ASH5 driven by the HCMV promoter and SCD4 and RZtr (Ribozyme against tat and rev) driven by the SV40 promoter and located in the 3'LTR.

### CONSTRUCTION OF PACKAGING CELL LINES

GP + env¹²⁰ cell line: 1 mcg pSV2gpt+10 mcg penv¹²⁰ where cotransfected into the env minus cell line (this cell line makes MLV gag and pol but not envelope) and the cells were put in HXM selection medium. 17 drug resistant colonies were isolated and expanded.

GP+env^{CD4} cell line: 1 mcg pSV2gpt + 10 mcg penv^{CD4} were cotransfected into the env minus cell line and the cells were subjected to HXM selection. 31 drug resistant colonies were isolated and expanded.

GP+env^{EPO} cell line: 1 mcg pSV2 gpt + 10 mcg penv^{EPO} were cotransfected into the env minus cell line. 21 HXM resistant colonies were isolated and enlarged.

All publications and patents mentioned in the above specification are herein incorporated by reference. The foregoing written specification is considered to be sufficient to enable one skilled in the art to practice the invention but is in no way limiting. Indeed, various modifications of the above-described modes for carrying out the invention may be made without departing from the spirit and scope of the present invention.

## Claims

1. An Expression Construct having at least one polynucleotide sequence derived from HIV genomic fragments oriented in antisense formation, and selected from the group consisting essentially of those substantially complementary to all or a portion of said genomic HIV:
i). in the env region, extending for about 3.4 kb from the 3' LTR of the HIV viral genome (about nts 5322-8694);
ii). in the gag region, extending about 2.8kb downstream from the 5' LTR of the HIV viral genome (about nts -342-2556);
iii). in the pol region, extending from about 2.8kb to about 4.5kb downstream from the 5' LTR of the HIV viral genome (about nts 2556-4227);
iv). in the pol region, extending from about 4.5kb to about 3.4kb upstream from the 3' LTR of the HIV viral genome (about nts 4227-5322); and,
v). in the gag region about nts 78-628.

2. An Expression Construct of claim 1 further comprising an endoribonuclease nucleotide sequence.

3. An Expression Construct of claim 2 wherein said endoribonuclease is the hammerhead sequence.

4. An expression construct in accordance with claim 1 wherein said HIV genomic fragment comprises the polynucleotide sequence of Figure 2.

5. An expression construct in accordance with claim 3 wherein said HIV genomic fragment and endoribonuclease sequence comprise the polynucleotide sequence of Figure 3.

6. An expression construct in accordance with claim 3 wherein said HIV genomic fragment and endoribonuclease sequence comprise the polynucleotide sequence of Figure 4.

7. A pharmaceutical composition comprising at least one expression construct of claim 1 and a pharmaceutically acceptable carrier or diluent.

8. A stably transformed cell comprising at least one expression construct of claim 1.

9. A retroviral vector comprising at least one expression construct of claim 1.

10. The retroviral vector of claim 9 wherein said expression construct comprises a polynucleotide sequence substantially complementary to a portion of HIV genomic RNA in the pol and tat region, about 1.1kb in length and situated from about 4.5kb to about 3.4kb upstream from the 3'LTR of the HIV genome.

11. The retroviral vector in accordance with claim 9 comprising an expression construct wherein said HIV genomic fragment is the polynucleotide sequence in Figure 2 or Figure 15 (SEQ.ID NO.:1 or SEQ.ID NO.:4).

12. A retroviral vector in accordance with claim 9 wherein said HIV genomic fragment and endoribonuclease sequence is the polynucleotide sequence in Figure 3 or Figure 16 (SEQ. ID NO.:2 or SEQ. ID NO.:5).

13. A retroviral vector in accordance with claim 9 comprising an expression construct wherein said HIV genomic fragment and endoribonuclease sequence is the polynucleotide sequence in Figure 4 or Figure 17 (SEQ. ID NO.:3 or SEQ. ID NO.:6).

14. The retroviral vector of claim 9 further comprising a genetic sequence operably disposed therein, and capable of encoding soluble CD4.

15. The retroviral vector of claim 14 wherein said sequence encoding soluble CD4 is operably disposed between U3 and U5 of the 3'LTR of said retroviral vector.

16. The retroviral vector of claim 15 wherein said expression construct is RZenv 1.

17. The retroviral vector of claim 15 wherein said expression construct is HXB5 (SEQ. ID. NO:1 or SEQ. ID NO:4).

18. The retroviral vector of claim 15 wherein said expression construct comprises a combination of RZenv1 (SEQ. ID NO: 2 or SEQ.ID NO: 5) and HXB5 (SEQ. ID NO: 1 or SEQ ID NO: 4) (SEQ.ID NO: 3 or SEQ. ID NO: 6).

19. The retroviral vector of claim 15 further comprising a foreign gene encoding a selectable marker.

20. The retroviral vector of claim 19 wherein said foreign gene is a bacterial neomycin resistant gene.

21. The retroviral vector of claim 20 further comprising a high efficiency, non-retroviral promoter.

22. The retroviral vector of claim 21 wherein said promoter is HCMV.

23. A pharmaceutical composition comprising the retroviral vector of any of claims 9 to 22 and a pharmaceutically acceptable carrier or diluent.

24. A recombinant retrovirus comprising at least one retroviral vector of any of claims 9 to 22.

25. A recombinant retrovirus capable of targeting and infecting cells that express a surface receptor for a select ligand, which comprises a retroviral vector of any of claims 9 to 22 and an exogenous genetic insert expressing said select ligand on the surface of said recombinant retrovirus.

26. The recombinant retrovirus of claim 25 wherein said select ligand is CD4.

27. The recombinant retrovirus of claim 25 wherein said select ligand is gp120.

28. A pharmaceutical composition comprising the recombinant retrovirus of any of claims 24 to 27 and a pharmaceutically acceptable carrier or diluent.

29. A vaccine useful for immunizing a patient against HIV infection which comprises a prophylactically effective amount of the retroviral vector of any of claims 9 to 22 in a pharmaceutically acceptable carrier or diluent.

30. A method for producing a foreign RNA which comprises:
i) transfecting a eucaryotic cell with a retroviral vector of claim 7 to stably transform said cell with said retroviral vector; and
ii) maintaining the resulting stably transformed cell under conditions permitting transcription of transcribable proviral DNA.

31. A method of producing a polypeptide comprising culturing the stably transformed cell of claim 30 under conditions permitting transcription of the proviral DNA into RNA and translation of the RNA into the polypeptide.

32. A method of producing a producer cell comprising the steps of:
i) transfecting a retroviral vector of any of claims 9 to 22 into a packaging cell line;
ii) culturing said cell line so transfected in suitable media and under conditions permitting transcription and translation of genetic material carried by said retroviral vector into a recombinant virion particle; and
iii) allowing said particles so formed to bud from said cell line into said media.

33. A method of preparing a composition for treating a patient with HIV infection, which method comprises:
a) removing cells from the patient; and
b) infecting the removed cells with an effective amount of the retroviral vector of any of claims 9 to 22.
